(19) Europäisches Patentamt
European Patent Office
Office européen des brevets

(11) **EP 1 990 408 A1**

(12) **EUROPEAN PATENT APPLICATION**
published in accordance with Art. 158(3) EPC

(43) Date of publication:
**12.11.2008 Bulletin 2008/46**

(51) Int Cl.:
*C12N 5/10* (2006.01)          *C12N 15/09* (2006.01)
*A61L 27/00* (2006.01)

(21) Application number: **07714071.3**

(22) Date of filing: **13.02.2007**

(86) International application number:
**PCT/JP2007/052489**

(87) International publication number:
**WO 2007/094302 (23.08.2007 Gazette 2007/34)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **14.02.2006 JP 2006036954**

(71) Applicants:
• **Senju Pharmaceutical Co., Ltd.**
**Osaka-shi,**
**Osaka 541-0046 (JP)**
• **Yamashita, Hidetoshi**
**Yamagata-shi, Yamagata 9902331 (JP)**

(72) Inventors:
• **YAMASHITA, Hidetoshi,**
**c/o 3-201, Yamagata University**
**Yamagata-shi, Yamagata 9902331 (JP)**

• **YAMAMOTO, Teiko**
**c/o Yamagata University School of Medicine**
**Yamagata-shi,**
**Yamagata 9909585 (JP)**
• **KASHIWAGI, Yoshiko,**
**c/o Yamagata University School of Medicine**
**Yamagata-shi, Yamagata 9909585 (JP)**

(74) Representative: **Duckworth, Timothy John**
**J.A. Kemp & Co.,**
**14 South Square,**
**Gray's Inn**
**London WC1R 5JJ (GB)**

(54) **SCLERAL CELL STRAIN**

(57)    The present invention provides a scleral cell strain capable of expressing an exogenous immortalizing gene, and a production method thereof. Since the scleral cell strain of the present invention can produce a sufficient number of cells and has constant and continuous proliferative capacity, it can be advantageously utilized for the elucidation of the pathogenesis of ophthalmic diseases such as scleral inflammation, and the development of a drug for the prophylaxis and/or treatment of said diseases. Moreover, the cell strain is not only highly useful for the biochemical· physiological studies of the sclera, and further for the study of cell differentiation mechanisms, but also possibly usable as a biological material of an artificial sclera.

EP 1 990 408 A1

## Description

## Technical Field

The present invention relates to a scleral cell strain, a production method thereof and the like.

## Background Art

Sclera is a membrane forming an external wall of the eyeball. Sclera looks white since it has a small number of blood vessels, and constitutes a part what is called the white of the eye. The sclera has a thickness of about 1 millimeter, and protects the eyeball together with the cornea, keeps a constant eyeball internal pressure and maintains the shape of the eyeball. The abnormality of the sclera causes various ophthalmic diseases (e.g., scleral inflammation, orbital inflammatory diseases, retinal vitreous diseases etc.), and decreases the eyesight. Particularly, remodeling of scleral extracellular matrix by cytokine and the like is highly important for the analysis of the pathology of such ophthalmic diseases and development of a therapeutic drug for the diseases.

Scleral cells are contained in the sclera, and produce a wide variety of scleral components such as collagen and the like. On the other hand, they are considered to be deeply involved in the remodeling of scleral extracellular matrix since they produce various degrading enzymes (matrix metalloproteinase etc.). Hence, it is important for the elucidation of the pathology of various ophthalmic diseases and advancement of the development of a therapeutic drug for the diseases to culture scleral cells in vitro and analyze their functions.

However, the proliferative capacity of scleral cells is considerably low to the extent that long-term culture is substantially impossible or, even if they could be cultured, the proliferation thereof is extremely difficult. In addition, scleral cells obtained from a neonate can be cultured rather easily but the proliferative capacity thereof is limited, and gigantism and denaturation of the cells may occur in a long-term passage. As such, the development of a scleral cell strain permitting long-term passage while maintaining the inherent functions of the scleral cell is desired.

In the meantime, a human crystalline epithelial cell line was produced by introducing a large T antigen gene of simian virus 40 (SV40) known to be an immortalizing gene into a human crystalline epithelial cell [JP-A-10-52272: patent reference 1, Andley U.P. et al., Invest. Ophthalmol. Vis. Sci., 35: 3094-3102 (1994): non-patent reference 1]. In addition, it has been reported that an immortalized cell line was produced by introducing E6 and E7 genes of papilloma virus [J. Virol., vol. 65, pp. 473-478 (1991): non-patent reference 2]. However, there has been no report on the establishment of scleral cell strain.

patent reference 1: JP-A-10-52272
non-patent reference 1: Invest. Ophthalmol. Vis. Sci., 35: 3094-3102 (1994)
non-patent reference 2: J. Virol., vol. 65, pp. 473-478 (1991)

## Disclosure of the Invention

## Problems to be Solved by the Invention

It is therefore an object of the present invention to provide a scleral cell strain useful for the physiological·biochemical studies of the sclera and as a research tool for the development of a drug for the prophylaxis or treatment of ophthalmic diseases such as scleral inflammation and the like, which can be transplanted into living organisms for regeneration of the sclera.

## Means of Solving the Problems

The present inventors have succeeded in conferring in vitro infinite proliferative capacity on scleral cells and establishing a clonal human scleral cell strain that can be passaged semipermanently, by infecting human scleral cells with a retrovirus vector functionally incorporating an immortalizing gene, which resulted in the completion of the present invention.

Accordingly, the present invention relates to the following.

[1] A scleral cell strain capable of expressing an exogenous immortalizing gene.
[2] The cell strain of [1], wherein the exogenous immortalizing gene encodes human papilloma virus E6 and E7.
[3] The cell strain of [1], which is derived from human.
[4] The cell strain of [1], which has the following properties (1) - (3):

(1) no decrease in the proliferation rate;
(2) substantial absence of gigantism·denaturation of cell; and
(3) increased expression of MMP1, MMP3 and HAS2 when stimulated with PDGF-BB.

[5] The cell strain of [4], further having the following property:

(4) increased expression of MMP3, HAS2 and HAS3 when stimulated with IL-1$\beta$.

[6] The cell strain of [4] or [5], wherein the above-mentioned properties are maintained for not less than 15 passages.
[7] The cell strain of [1], which is established by transfecting an expression vector functionally harboring an exogenous immortalizing gene into scleral cells and passaging the cells in a medium.

[8] A production method of a scleral cell strain, comprising transfecting an expression vector functionally harboring an exogenous immortalizing gene into scleral cells and passaging the cells in a medium.

[9] The production method of [8], wherein the exogenous immortalizing gene encodes human papilloma virus E6 and E7.

**Effect of the Invention**

Since the scleral cell strain of the present invention can produce a sufficient number of cells and has constant and continuous proliferative capacity, it can be advantageously utilized for the elucidation of the pathogenesis of ophthalmic diseases such as scleral inflammation, and the development of a drug for the prophylaxis and/or treatment of said diseases. Moreover, the cell strain is not only highly useful for the biochemical·physiological studies of the sclera, and further for the study of cell differentiation mechanisms, but also possibly usable as a biological material of an artificial sclera.

**Brief Description of the Drawings**

Fig. 1 is a photograph showing the morphology of a scleral cell strain.
Fig. 2 schematically shows examination protocol.
Fig. 3 shows the effect of PDGF-BB and/or TGF-β1 stimulation on MMP1, 2 and 3, TIMP1 and 2, and Has1, 2 and 3 expressions by a scleral cell strain.
Fig. 4 schematically shows examination protocol.
Fig. 5 shows the effect of PDGF-BB or IL-1β stimulation on HAS1, 2 and 3, CD44, MMP1, 2 and 3, and TIMP1 and 2 expressions by a scleral cell strain. 1: control (0 hr), 2: PDGF-BB (6 hr), 3: PDGF-BB (24 hr), 4: IL-1β (6 hr), 5: IL-1β (24 hr).
Fig. 6 schematically shows examination protocol.
Fig. 7 shows the effect of hydrocortisone treatment on HAS1, 2 and 3, CD44, MMP1, 2 and 3, and TIMP 1 and 2 expression by a scleral cell strain. 1: control, 2: EtOH (0.1%), 3: EtOH (1%), 4: hydrocortisone (10 μg/ml), 5: hydrocortisone (100 μg/ml).

**Best Mode for Carrying out the Invention**

The scleral cell strain of the present invention is a cell group containing an exogenous immortalizing gene, which has acquired infinite in vitro proliferative capacity as a result of the expression of the immortalizing gene.

The scleral cell strain of the present invention is derived from a mammal. Examples of the mammal include rodents such as mouse, rat, hamster, guinea pig and the like, experiment animals such as rabbit and the like, artiodactyls such as swine, bovine, goat, sheep and the like, perissodactyls such as horse and the like, pets such as dog, cat and the like, primates such as human, monkey, Macaca mulatta, marmoset, orangutan, chimpanzee and the like; and the like. The scleral cell strain of the present invention is preferably derived from human.

Here, the "immortalizing gene" refers to a gene that immortalizes cells and confers infinite proliferative capacity on the cells. Examples thereof include oncogenes such as c-myc, ras and the like, adenovirus E1A, SV40 derived large T antigen gene (SV40 large T antigen gene), large T antigen gene of polyoma virus, E6 and E7 genes of papilloma virus, and the like. In the present invention, the "exogenous immortalizing gene" means an immortalizing gene newly introduced extracellularly, which is not inherently possessed by the scleral cell to be the origin of the scleral cell strain of the present invention. Therefore, the exogenous immortalizing gene includes, in addition to an immortalizing gene derived from a source other than the mammal from which the cell strain of the present invention derives, for example, even an oncogene of the mammal from which the cell strain of the present invention derives, which has been modified into a form that permits expression in a target cell (i.e., free of suppression of expression that normal endogenous oncogenes are under). As the exogenous immortalizing gene of the present invention, a virus-derived immortalizing gene, more preferably E6 and E7 genes of papilloma virus, are used. Using E6 and E7 genes of papilloma virus, scleral cells can be immortalized highly efficiently as compared to the use of SV40 large T antigen gene and the like.

The scleral cell strain of the present invention can be preferably established by transfecting scleral cells separated from a tissue with an expression vector functionally harboring the above-mentioned exogenous immortalizing gene, namely, harboring the gene in a form allowing expression in a target cell, and passaging the cells in a suitable medium.

The scleral cells can be obtained by, for example, separating a scleral tissue from an isolated eye tissue, cutting it small (about 1 mm square) with scissors etc., and digesting the obtained scleral fragments with type IV collagenase. The scleral cells are preferably scleral parenchymal cells. The scleral cells are suspended in a suitable liquid medium, for example, Eagle MEM medium, Dulbecco modified Eagle MEM medium, Ham-F12 medium, Katsuta medium DM-160 and the like containing 10 - 20% of fetal bovine serum or calf serum, and cultured in a $CO_2$ incubator for about ) 2 days. Cultured scleral cells are subjected to transfection.

The expression vector to be used in the present invention is exemplified by plasmid vectors and virus vectors. When a virus genome is used as a vector, it is more preferable to delete or mutate the gene partially so that at least complete virus particles will not be produced in a cell harboring the vector.

As a construction method of an expression vector functionally harboring an exogenous immortalizing gene when, for example, an E6 and E7 of papilloma virus gene is used as an exogenous immortalizing gene, the following method can be mentioned. A DNA fragment containing an immortalizing gene present in the early region of genomic DNA of human papilloma virus (i.e., E6 and E7 genes) is cleaved out with a suitable restriction enzyme, and inserted in an expression vector containing a promoter capable of expressing the gene in a scleral cell. Examples of the promoter include SV40 early promoter, Rous sarcoma virus (RSV) promoter, MuMLV LTR and the like.

Examples of the plasmid vector include pBR322, pGEM and the like. Moreover, a transcription termination signal, a specific sequence that enhances the expression of an exogenous immortalizing gene and the like may be provided at a suitable site of the vector. The thus-constructed exogenous immortalizing gene expression vector is synthesized in a large amount in a suitable host, such as Escherichia coli, yeast, Bacillus subtilis etc., recovered and purified by a conventional method, and introduced into scleral cells by a conventional gene transfer method, such as calcium phosphate coprecipitation method, microinjection method, polyethylene glycol method, electroporation method and the like.

Examples of the virus vector include retroviruses such as moloney murine leukemia virus, lentivirus and the like, adenovirus, herpesvirus, adeno-associated virus, parvovirus, semliki forest virus, vaccinia virus, Hemagglutinating Virus of Japan and the like. Transgene by a retrovirus is preferable since the gene is introduced by incorporation into a chromosome. In addition, lentivirus can infect both dividing and non-dividing cells to introduce a gene. An exogenous immortalizing gene is introduced into scleral cells by culturing the scleral cells in a culture medium containing virus particles. In this case, the introduction efficiency can be increased by using RetroNectin, fibronectin, polybrene and the like as a gene transfer reagent.

The cells after transfection are cultured in a liquid medium such as Eagle MEM medium, Dulbecco modified Eagle MEM medium, Ham-F12 medium, Katsuta medium DM-160 and the like. As the conditions of culture temperature, medium pH, $CO_2$ concentration and the like, general conditions conventionally used for culturing animal cells can be appropriately employed. The medium is exchanged with a fresh medium every 2 - 3 days, and when the cell proliferation becomes saturated, the cells are passaged. Even after termination of cell proliferation, the culture is continued and the group of cells that has started to grow again is divided as a monoclonal cell group. The clones can be separated as follows. A filter paper having a small diameter, which has been soaked with trypsin and EDTA solution, is placed still on the objective clonal cell group, and the cells are detached from a culture vessel and attached to the filter paper. The clonal cell group attached to the small filter paper piece is placed in a different culture vessel together with the filter paper. The clones can also be separated by a method of picking up the colony, a limiting dilution method or a method using a cell sorter. Since respective cells of a clonal cell strain show uniform properties, they can be useful model cells for physiological· biochemical studies of the scleral body, elucidation of pathogenesis of retinal scleral diseases, development of a prophylactic or therapeutic drug for ophthalmic diseases such as scleral inflammation and the like, which have conventionally been performed using heterogeneous scleral cells.

The scleral cell strain obtained as mentioned above can be passaged by a general animal cell cultivation technique. As an index of proliferative capacity of cell, a cell population doubling level (PDL) calculated by the following formula can be used.

$$PDL = log_{10}(N_i / N_{0)}) / log_{10} 2$$

$$PDL = log_{10}(N_i / N_0) / log_{10} 2$$

$N_i$ = number of cells at completion of the ith passage culture
$N_0$ = number of cells at start of the first passage culture
The PDL of the cell strain of the present invention generally increases generally by about 1.1 - 2.0, preferably 1.4 - 1.5, per one passage.

The cell strain of the present invention can have the following properties (1) - (3):

(1) no decrease in the proliferation rate;
(2) substantial absence of gigantism·denaturation of cell; and
(3) increased expression of MMP1, MMP3 and HAS2 when stimulated with PDGF-BB.

Being "substantially free of gigantism·denaturation of cells" means that partially elongated cells may be found by a microscopic observation of the cells, but the majority (for example, about 95% or more) thereof shows the inherent morphological characteristics of a scleral cell. MMP1 is collagenase capable of degrading type I, III collagens. MMP3 is a protease capable of degrading many extracellular matrices such as type IV collagen, laminin and the like as substrates. HAS2 (Hyaluronan synthase 2) is a synthase of hyaluronic acid. The "gene expression" means expression of mRNA encoding the gene or a gene product (protein) thereof. Increased expression of MMP1, MMP3 and HAS2 by stimulation with PDGF-BB is an index of the physiological characteristics as a scleral cell possessed by the cell strain of the present invention. It is considered that remodeling of scleral extracellular matrix is involved in various ophthalmic diseases, and various degrading enzymes (matrix metalloproteinase etc.) and synthases (HAS2 etc.) are involved in the remodeling. Accordingly, the cell strain of the present invention is useful as a powerful tool particularly for studying the involvement of various degrading enzymes and synthases released from scleral cells due to cytokine stimulation in the remodeling of scleral extracellular matrix and various ophthalmic diseases.

The cell strain of the present invention may further have, in addition to the above-mentioned properties, the following property (4).

(4) Increased expression of MMP3, HAS2 and HAS3 when stimulated with IL-1β.

Increased expression of MMP3, HAS2 and HAS3 by stimulation with IL-1β is also index of the physiological characteristics as a scleral cell possessed by the cell strain of the present invention. Accordingly, the cell strain of the present invention is useful as a powerful tool particularly for studying the involvement of MMP3, HAS2 and HAS3 released from scleral cells due to IL-1β stimulation in the remodeling of scleral extracellular matrix and various ophthalmic diseases.

In the scleral cell strain of the present invention, the above-mentioned properties are stably maintained for 15 passages or more, preferably 20 passages or more.

The cell strain of the present invention can be a useful model cell for the physiological·biochemical studies of the sclera, elucidation of pathogenesis of retinal scleral diseases and development of a prophylactic or therapeutic drug for the diseases, as well as safety tests of pharmaceutical products for ophthalmic diseases, and the like. In addition, since stable supply of the cell is possible, it can be a useful material for transplantation aiming at scleral regeneration and an artificial vitreous body. Moreover, a papilloma virus antigen transfected cell strain can be subjected to cell biological experiments.

The present invention is explained in more detail in the following by referring to Examples, which are not to be construed as limitative.

[Example 1]

(Preparation of human scleral cells)

The scleral tissue was isolated from the eyeball of a 65-year-old female affected with malignant melanoma and removed to excise the affected part. The sclera tissue was cut into 1 mm square fragments with scissors. The obtained sclera tissue fragments were digested in 0.05% type IV collagenase/DMEM at 37°C for 16 hr. The scleral cells recovered from the digestion product were suspended in DMEM (GIBCO) (10% FBS), and cultured at 37°C, 5% $CO_2$. This experiment was approved by the Yamagata University Faculty of Medicine Ethics Committee. A written approval was obtained from the above-mentioned patient before the experiment.

(Preparation of packaging cell PA317 harboring human papilloma virus type 16 E6 and E7 and preparation of virus stock)

PA317 (mouse packaging cell: ATCC CRL-2203) containing pLXSN vector harboring human papilloma virus type 16 E6 and E7 was cultured in DMEM (GIBCO) (10% FBS, high glucose) at 37°C, 5% $CO_2$. The cells were cultured up to 90% confluent, and the culture supernatant was recovered as a virus stock and preserved at -80°C until use.

(RetroNectin coating)

1 mg/ml of RetroNectin (Wako #T100A) was diluted with PBS (pH 7.2) to 25 μg/ml, and added into a 35 mm culture dish by 2 ml. The dish was stood in a clean bench at room temperature for 2 hr for RetroNectin coating. Thereafter, 2% BSA/PBS (2 ml) was added for blocking, the blocking solution was washed with 2 ml of PBS, and PBS was removed.

(Infection of scleral cells with retrovirus)

The above-mentioned virus stock solution (2.5 ml) was added to a 35 mm culture dish coated with RetroNectin, and stood at 37°C, 5% $CO_2$ for 4 - 6 hr to bind RetroNectin to a retrovirus vector. Thereafter, the virus stock solution was removed, and the dish was washed with PBS. The scleral cells were added at concentration of $0.5 - 1.0 \times 10^5$ cells/2 ml DMEM (10% FBS)/35 mm dish, and cultured at 37°C, 5% $CO_2$ for 2 days.

(Isolation of scleral cell strain)

The scleral cells infected with retrovirus was passaged into a 10 mm dish, and further passaged for 5 days. The formed colony was picked up, transferred into a 24 well plate, and the colony was cultivated to establish a monoclonal human scleral cell strain.

(Passage of cells)

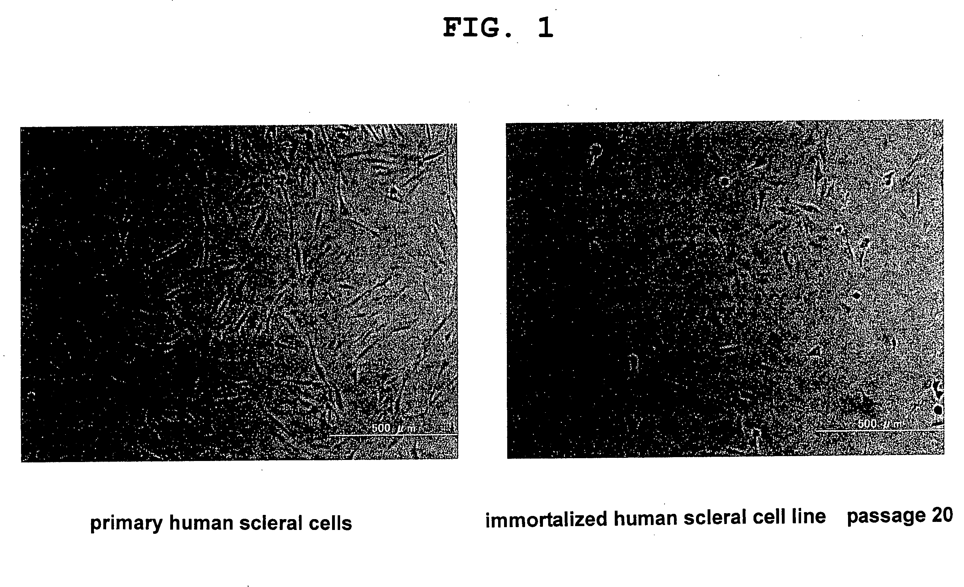

The isolated colonies were continuously cultured until the 20th passage under similar conditions. The cells could be continuously passaged at a constant ratio throughout the culture, and therefore, the cell proliferation rate did not decrease. PDL was calculated every 5 passages. The PDL of the obtained cell strain increased by about 1.4 - 1.5 for every passage.
Cell morphology was observed under a microscope. The appearance of the cells was photographed with a cooling CCD camera DP-70 (manufactured by OLYMPUS) connected to a DMIRBE microscope (manufactured by Leica). The photographing conditions were as follows: objective 5-fold/ocular 10-fold/quantity of light 9.0 V. The cells consisted of a single layer with a fibroblast-like morphology (Fig. 1). Gigantism and denaturation of the cells were not observed.

(Confirmation of human papilloma virus type 16 E6 and E7 expression)

Total RNA was isolated from the cells, cDNA was synthesized, and the mRNA expression of immortalizing gene human papilloma virus type 16 E6 and E7 was confirmed by the RT-PCR method.

(Analysis of scleral cell strain phenotype)

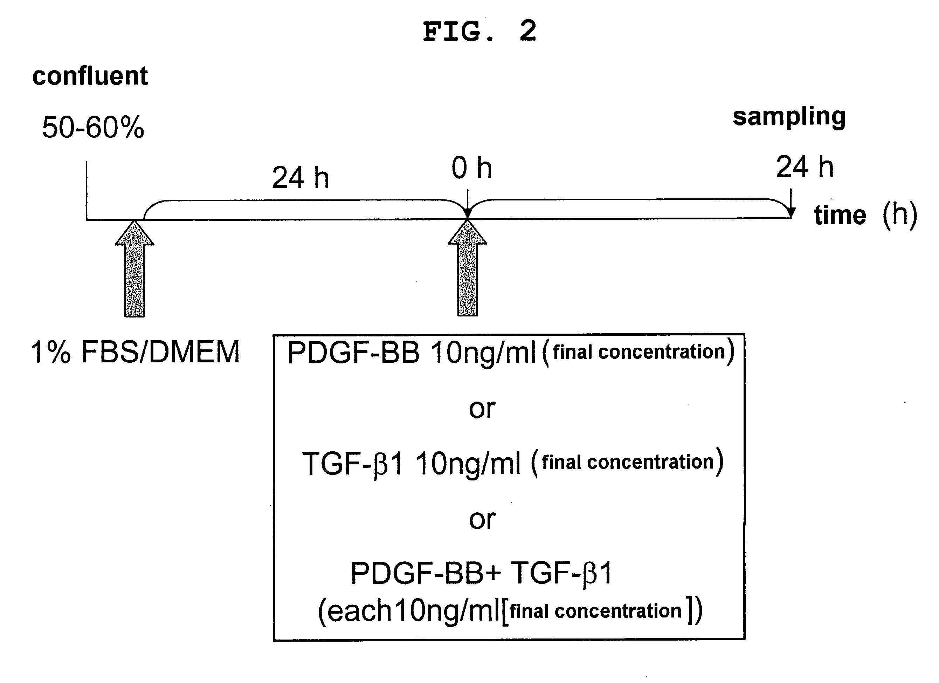

The effect of PDGF-BB and/or TGF-β1 on expression of hyaluronic acid synthase (HAS) 1, 2 and 3, matrix metalloproteinase (MMP) 1, 2 and 3, and tissue inhibitor of metalloproteinases (TIMP) 1 and 2 in the established vitreous cell strain were examined. The expression was observed by RT-PCR.
To be specific, when the scleral cell strain was 60 - 70% confluent, the medium of the cells was exchanged with DMEM containing 1% bovine serum and, after culturing for 24 hr, the cells were stimulated with TGF-β1 (10 ng/ml: final concentration) and/or PDGF-BB (10 ng/ml: final concentration), and were further cultured for 24 hr. The cells were recovered at 24 hr after the cytokine stimulation, and total RNA was extracted, cDNA was synthesized, and RT-PCR was performed using primers specific to MMP1, 2 and 3, TIMP1 and 2 and HAS1, 2 and 3 (Fig. 2). Using Roche FastStart High Fidelity PCR System, PCR was performed on Gene Amp PCR System 9700 (Applied Biosystems). The PCR conditions were as follows:

95°C 2 min. → (95°C 30 seconds → X°C 30 seconds → 72°C 30 seconds) ×Y cycles → 72°C 7 min
X=50, Y=35···HAS1, 2 and 3, MMP2 and 3
X=60, Y=30···MMP1, TIMP2
X=50, Y=30···TIMP1
X=60, Y=25···beta-Actin

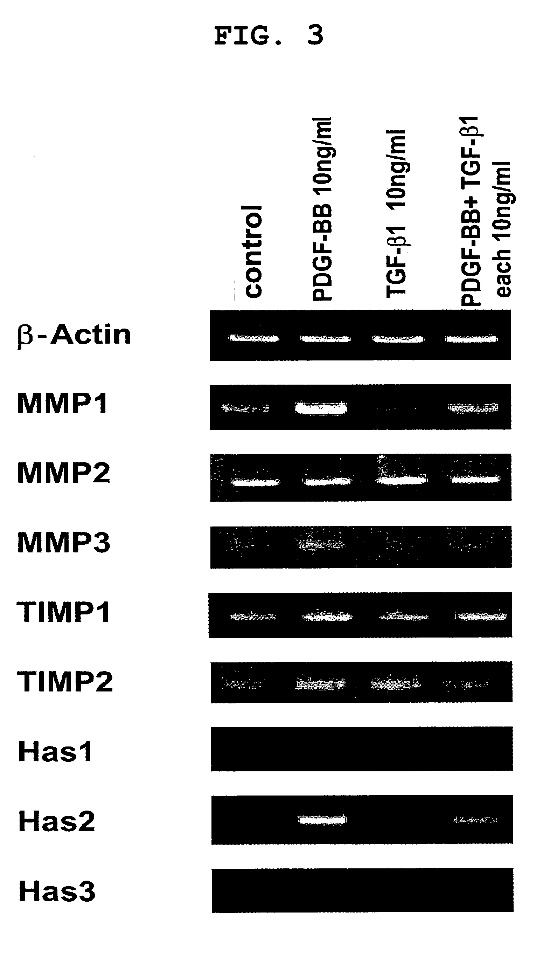

As a result, PDGF-BB induced mRNA expression of MMP1, MMP3 and HAS2. TGF-β1 hardly influenced expression of the tested gene. A cross talk (a synergistic effect) between PDGF-BB and TGF-β1 was not observed (Fig. 3).
The above shows that the obtained scleral cell strain maintained physiological characteristics as a scleral cell.

[Example 2]

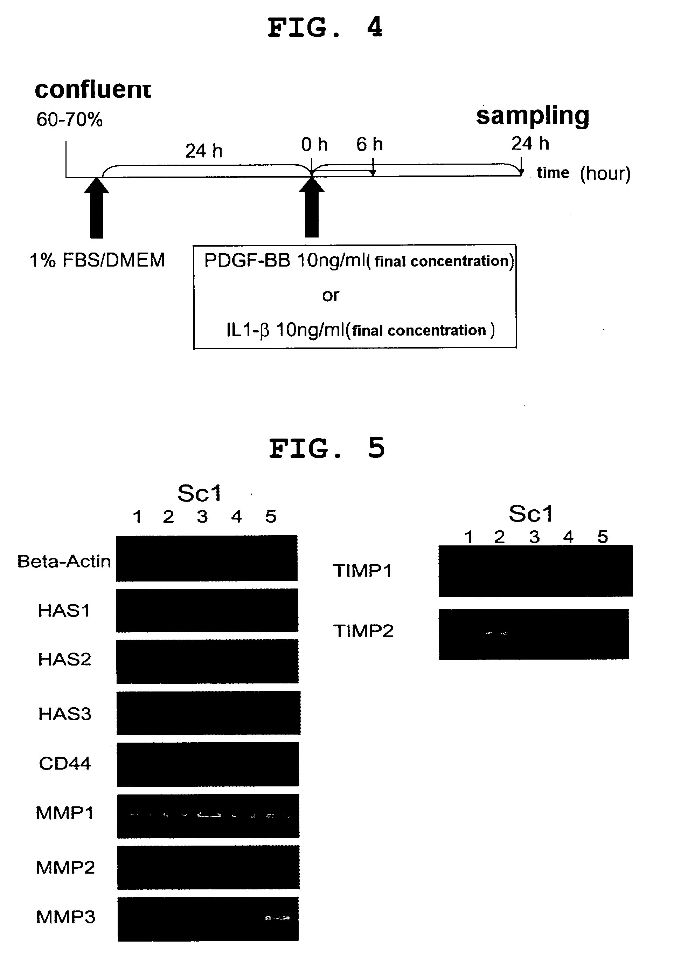

The effect of PDGF-BB (10 ng/ml) or IL-1β (10 ng/ml) on the expression of hyaluronic acid synthases (HAS) 1, 2 and 3, CD44, matrix metalloproteinases (MMP) 1, 2 and 3, and tissue inhibitor of metalloproteinases (TIMP) 1 and 2 in the scleral cell strain established in Example 1 was examined (Fig. 4). The expression was observed by RT-PCR. The conditions of culture and RT-PCR followed those in Example 1.

As a result, IL-1β induced mRNA expression of MMP3 and HAS 2, 3 (Fig. 5).

[Example 3]

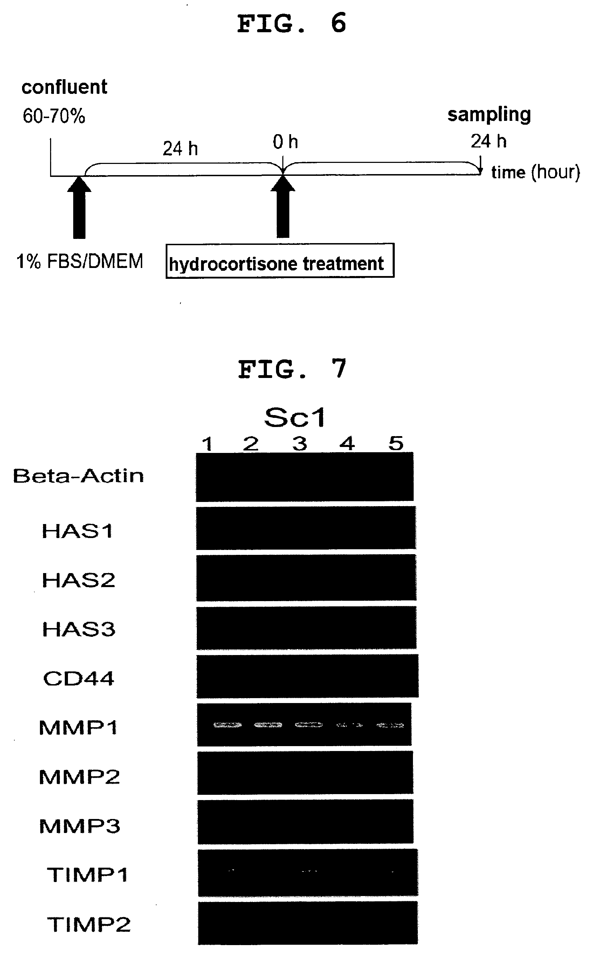

The effect of hydrocortisone on the expression of hyaluronic acid synthases (HAS) 1, 2 and 3, CD44, matrix metalloproteinases (MMP) 1, 2 and 3, and tissue inhibitor of metalloproteinases (TIMP) 1 and 2 in the scleral cell strain established in Example 1 was examined. To be specific, when the cells were 60 - 70% confluent, the medium for the cells was exchanged with DMEM containing 1% bovine serum and, after culturing for 24 hr, the cells were treated with hydrocortisone (10 or 100 μg/ml) and further cultured for 24 hr (Fig. 6). Thereafter, the cells were recovered, total RNA extraction and cDNA synthesis were performed, and RT-PCR was conducted using various genes. The conditions of RT-PCR followed those in Example 1.
As a result, an influence of the hydrocortisone treatment on the mRNA expression of various genes was not observed (Fig. 7). The results show that the expression of the above-mentioned respective genes by the cell strain of the present invention is hardly influenced by hydrocortisone. In addition, the possibility was suggested that a steroid hormone causes no rapid change of extracellular matrix. Therefrom it is considered that the extracellular matrix of scleral cells (tissue) is not influenced by a temporary administration of a steroid hormone used for the treatment.

**Industrial Applicability**

Since the scleral cell strain of the present invention can produce a sufficient number of cells and has constant and continuous proliferative capacity, it can be advantageously utilized for the elucidation of the pathogenesis of ophthalmic diseases such as scleral inflammation, and the development of a drug for the prophylaxis and/or treatment of said diseases. Moreover, the cell strain is not only highly useful for the biochemical·physiological studies of the sclera, and further for the study of cell differentiation mechanisms, but also possibly usable as a biological material of an artificial sclera.
This application is based on a patent application No. 2006-036954 filed in Japan (filing date: February 14, 2006), the contents of which are incorporated in full herein by this reference.

**Claims**

1. A scleral cell strain capable of expressing an exogenous immortalizing gene.

2. The cell strain of claim 1, wherein the exogenous immortalizing gene encodes human papilloma virus E6 and E7.

**3.** The cell strain of claim 1, which is derived from human.

**4.** The cell strain of claim 1, which has the following properties (1) - (3):

(1) no decrease in the proliferation rate;
(2) substantial absence of gigantism·denaturation of cell; and
(3) increased expression of MMP1, MMP3 and HAS2 when stimulated with PDGF-BB.

**5.** The cell strain of claim 4, further having the following property:

(4) increased expression of MMP3, HAS2 and HAS3 when stimulated with IL-1β.

**6.** The cell strain of claim 4 or 5, wherein the abovementioned properties are maintained for not less than 15 passages.

**7.** The cell strain of claim 1, which is established by transfecting an expression vector functionally harboring an exogenous immortalizing gene into scleral cells and passaging the cells in a medium.

**8.** A production method of a scleral cell strain, comprising transfecting an expression vector functionally harboring an exogenous immortalizing gene into scleral cells and passaging the cells in a medium.

**9.** The production method of claim 8, wherein the exogenous immortalizing gene encodes human papilloma virus E6 and E7.

FIG. 1

primary human scleral cells

immortalized human scleral cell line    passage 20

# FIG. 2

confluent

50-60%

sampling

24 h       0 h       24 h

time (h)

1% FBS/DMEM

PDGF-BB 10ng/ml (final concentration)

or

TGF-β1 10ng/ml (final concentration)

or

PDGF-BB+ TGF-β1
(each10ng/ml[final concentration])

EP 1 990 408 A1

# FIG. 3

# FIG. 4

**confluent**
60-70%

**sampling**

24 h     0 h    6 h      24 h

time (hour)

1% FBS/DMEM

| PDGF-BB 10ng/ml( final concentration) |
| --- |
| or |
| IL1-β 10ng/ml(final concentration ) |

# FIG. 5

Sc1
1 2 3 4 5

Beta-Actin

HAS1

HAS2

HAS3

CD44

MMP1

MMP2

MMP3

Sc1
1 2 3 4 5

TIMP1

TIMP2

# FIG. 6

confluent
60-70%

sampling

24 h     0 h     24 h

time (hour)

1% FBS/DMEM    hydrocortisone treatment

# FIG. 7

Sc1

1 2 3 4 5

Beta-Actin

HAS1

HAS2

HAS3

CD44

MMP1

MMP2

MMP3

TIMP1

TIMP2

## INTERNATIONAL SEARCH REPORT

| | International application No. |
|---|---|
| | PCT/JP2007/052489 |

| A. CLASSIFICATION OF SUBJECT MATTER |
|---|
| *C12N5/10*(2006.01)i, *C12N15/09*(2006.01)i, *A61L27/00*(2006.01)n |
| |
| According to International Patent Classification (IPC) or to both national classification and IPC |

| B. FIELDS SEARCHED |
|---|
| Minimum documentation searched (classification system followed by classification symbols) |
| C12N5/10, C12N15/09, A61L27/00 |
| |
| Documentation searched other than minimum documentation to the extent that such documents are included in the fields searched |
| |
| Electronic data base consulted during the international search (name of data base and, where practicable, search terms used) |
| BIOSIS/MEDLINE/WPIDS(STN), CA(STN), JSTPlus(JDream2) |

| C. DOCUMENTS CONSIDERED TO BE RELEVANT |
|---|

| Category* | Citation of document, with indication, where appropriate, of the relevant passages | Relevant to claim No. |
|---|---|---|
| X<br>Y | Cambrey AD et al., "Characterization of a standard strain of humanscleral fibroblasts.", ARVO Annual Meeting Abstract Search andProgram Planner (2003) Vol.2003, p. Abstract No.1199.cd-rom, Meeting Info: Annual Meeting of the Association for Research inVision and Ophthalmology, Fort Lauderdale, FL, USA, May 04-08, 2003, Association for Research in Vision and Ophthalmology(Meeting Abstract), BIOSIS onlineretrieved on 11 April 2007]BIOSIS Accession no. 2003:529309 | 1-8<br>9 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 20 April, 2007 (20.04.07) | 01 May, 2007 (01.05.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

## INTERNATIONAL SEARCH REPORT

International application No.

PCT/JP2007/052489

C (Continuation).   DOCUMENTS CONSIDERED TO BE RELEVANT

| Category* | Citation of document, with indication, where appropriate, of the relevant passages | Relevant to claim No. |
|---|---|---|
| X | ENGELBRECHT-SCHNUR S et al., "Dexamethasone treatment decreaseshyaluronan-formation by primate trabecular meshwork cells invitro", Exp. Eye. Res. (1997) Vol.64, No.4, p.539-543 | 1-3 |
| Y | HALBERT CL et al., "The E7 gene of human papillomavirus type 16is sufficient for immortalization of human epithelial cells", J. Virol. (1991) Vol.65, No.1, p.473-478 | 9 |
| Y | HARVEY HA et al., "Immortalization of human urethral epithelialcells: a model for the study of the pathogenesis of and theinflammatory cytokine response to Neisseria gonorrhoeaeinfection", Infect. Immun. (2002) Vol.70, No.10, p.5808-5815 | 9 |
| Y | AKIMOV SS et al., "Bypass of senescence, immortalization, andtransformation of human hematopoietic progenitor cells." StemCells (2005) Vol.23, No.9, p.1423-1433 | 9 |
| P,X<br>P,Y | Yoshiko KASHIWAGI et al., "Kyomaku ni Okeru Saibo Gaikishitsu Sansei Seigyo to Remodeling no Bunshi Byotai", Dai 110 Kai Annual Meeting of the Japanese Ophthalmological Society, Koen Shoroku, 15 March, 2006 (15.03.06), p.210 | 1-8<br>9 |
| P,X | QU J et al., "The presence of m1 to m5 receptors in human sclera:evidence of the sclera as a potential site of action formuscarinic receptor antagonists", Curr. Eye Res. 2006 Jul-Aug, Vol.31, No.7-8, p.587-597 | 1-3 |
| A | WESTERGREN-THORSSON G et al., "The synthesis of a family ofstructurally related proteoglycans in fibroblasts isdifferently regulated by TFG-beta", Matrix (1991) Vol.11, No.3, p.177-183 | 1-9 |
| A | WATANABE K et al., "Scleral fibroblasts of the chick embryoproliferate by an autocrine mechanism in protein-free primarycultures: differential secretion of growth factors depending onthe growth state", Exp. Cell Res. (1989) Vol.182, No.2, p.321-329 | 1-9 |

Form PCT/ISA/210 (continuation of second sheet) (April 2005)

## REFERENCES CITED IN THE DESCRIPTION

**Patent documents cited in the description**

- JP 10052272 A

- JP 2006036954 A

**Non-patent literature cited in the description**

- **ANDLEY U.P. et al.** *Invest. Ophthalmol. Vis. Sci.,* 1994, vol. 35, 3094-3102
- *J. Virol.,* 1991, vol. 65, 473-478

- *Invest. Ophthalmol. Vis. Sci.,* 1994, vol. 35, 3094-3102